# DEMANDE DE BREVET EUROPEEN

(11) **EP 4 772 066 A1**
(43) Date de publication de la demande: **08.07.2026**
(21) Numéro de dépôt: 25221954.8
(22) Date de dépôt: 09.12.2025
(51) Int. Cl.: A45C 5/06, A45C 7/00, A61B 50/31, A61F 17/00, A61M 16/00

(54) **SACOCHE DE TRANSPORT DE VENTILATEUR MÉDICAL**

(30) Priorité: 02.01.2025 FR 2500016
(71) Demandeur: Air Liquide Medical Systems, 92182 Antony Cedex (FR)
(72) Inventeur: PETIT, Christophe, 92182 Antony Cedex (FR); PODEVIN, Johanna, 92182 Antony Cedex (FR)
(74) Mandataire: Air Liquide

(57) **Abrégé**

L'invention concerne une sacoche de transport (1) pour ventilateur médical (50) comprenant un corps de sacoche (2) comprenant une paroi avant (20) et définissant un compartiment interne (4) configuré et dimensionné pour loger un ventilateur médical (50). La paroi avant (20) du corps de sacoche (2) comprend au moins une fenêtre principale (5) comprenant une première vitre (6) transparente et un panneau de protection (3) comprenant une seconde vitre (7) transparente, est fixé de manière pivotante au corps de sacoche (2) pour venir se positionner face à la fenêtre principale (5) lorsqu'il est dans une position repliée (P1). Optionnellement, un sac à accessoires (60) détachable peut être fixé à une paroi arrière (21) du corps de sacoche (2).

## Description

L'invention concerne une sacoche de transport d'un ventilateur médical comprenant un panneau de protection avant amovible et pivotant équipé d'une vitre transparente en polymère, de préférence rigide, lequel vient protéger une vitre transparente en polymère, de préférence souple, agencée sur le corps de sacoche.

Les ventilateurs médicaux sont des appareils d'assistance respiratoire servant à administrer un gaz, tel de l'air, de l'oxygène ou un mélange air/oxygène, à une personne en ayant besoin, typiquement un patient.

Lorsqu'il est destiné à être utilisé par un service d'urgence, tels les pompiers, les SAMU ou analogue, un ventilateur doit pouvoir être transporté sur site tout en étant protégé contre les chocs, les détériorations, les salissures, etc..

Ainsi, EP3791911 propose un ventilateur médical protégé par un exosquelette rigide, agencé autour du ventilateur, qui le protège contre les chocs. Toutefois, cet exosquelette ne permet pas de le protéger contre les salissures, les intempéries (pluie, neige, vent...), en particulier l'écran d'affichage équipant le ventilateur, lequel écran est particulièrement fragile. De plus, un tel exosquelette ajoute un poids non-négligeable à l'ensemble, ce qui n'est pas souhaitable.

Une autre solution connue consiste à insérer le ventilateur médical dans une sacoche de transport. Or, dans la plupart des cas, il convient d'extraire le ventilateur médical de la sacoche afin de pouvoir l'utiliser, ce qui l'expose alors à nouveau aux détériorations, aux intempéries...

DE102007016410 propose une enceinte de transport pour loger un défibrillateur et un ventilateur médical, qui est dotée en façade, d'une fenêtre avec vitre transparente. Cette solution n'est pas idéale car la vitre en façade peut être salie ou détériorée pendant son utilisation, ce qui peut nuire à une bonne lecture des informations affichées sur l'un ou l'autres des appareils, obligeant alors l'utilisateur à ouvrir le panneau vitré avec le risque d'exposer lesdits appareils aux détériorations, aux intempéries....

Un problème est dès lors de pouvoir transporter et protéger un ventilateur médical sans rencontrer tout ou partie des problèmes susmentionnés.

Une solution de l'invention concerne alors une sacoche pour ventilateur médical comprenant un corps de sacoche comprenant une paroi avant et définissant un compartiment interne configuré et dimensionné pour loger un ventilateur médical.

La paroi avant du corps de sacoche comprend au moins une fenêtre principale comprenant une première vitre transparente, de préférence une vitre transparente souple.

De plus, un panneau de protection, i.e. panneau avant, aussi appelé écran de protection, bouclier ou analogue, comprenant une seconde vitre transparente, de préférence une vitre transparente rigide, est fixé de manière pivotante au corps de sacoche de manière à venir se positionner face à la fenêtre principale lorsqu'il est dans une position repliée (P1).

Selon le mode de réalisation considéré, la sacoche selon l'invention peut comprendre l'une ou plusieurs des caractéristiques suivantes :
- la fenêtre principale comprend une ouverture pratiquée dans la paroi avant du corps de sacoche et recouverte par la première vitre transparente, c'est-à-dire que la première vitre transparente est logée et/ou fixée dans l'ouverture de la fenêtre principale de sorte de l'obturer ou de la recouvrir totalement.
- le panneau de protection est agencé extérieurement à la sacoche, i.e. face à la fenêtre principale lorsqu'il est dans une position repliée.
- la fenêtre principale comprenant la première vitre transparente souple est située, i.e. prise en « sandwich », entre le panneau de protection avant et le compartiment interne de la sacoche, lorsque le panneau de protection est en position replié (position P1).
- la fenêtre principale comprend une ouverture carrée d'environ 16 à 20 cm de côté. Bien entendu, elle pourrait avoir d'autres dimensions.
- le panneau de protection (i.e. écran de protection avant) comprend un rebord ou une bordure périphérique agencée autour de la seconde vitre transparente.
- la seconde vitre présente une rigidité supérieure à celle de la première vitre, c'est-à-dire que la première vitre est plus souple ou flexible que la seconde vitre.
- la bordure périphérique du panneau de protection avant est en polymère, par exemple en un tissé polymère ou tout autre matériau adapté.
- la première vitre transparente, de préférence souple, et la seconde vitre transparente, de préférence rigide, sont en polymère.
- la première vitre transparente, dite « vitre souple », est plus souple ou plus flexible que la seconde vitre transparente, dite « vitre rigide ».
- la première vitre transparente et la seconde vitre transparente sont formées de polymères identiques ou différents, de préférence différents l'une de l'autre.
- la première vitre transparente comprend préférentiellement un polymère de type polychlorure de vinyle ou PVC, typiquement une feuille souple de PVC.
- la seconde vitre transparente comprend préférentiellement un polymère de type polytéréphtalate d'éthylène ou PET, typiquement une feuille ou plaque rigide de PET.
- l'épaisseur de la première vitre transparente, i.e. vitre souple, est inférieure à l'épaisseur de la seconde vitre transparente, i.e. vitre rigide.
- l'épaisseur de la première vitre transparente souple est de préférence inférieure à 1 mm, typiquement inférieure à 0,8 mm.
- l'épaisseur de la seconde vitre transparente rigide est de préférence supérieure à 1mm, de préférence de plusieurs mm, typiquement entre 2 et 20 mm.
- la première vitre transparente souple est une feuille, une plaque fine ou analogue.
- la seconde vitre transparente rigide est une plaque, une feuille épaisse ou analogue.
- les première et seconde vitres transparentes sont (quasi)planes.
- le corps de sacoche est souple et/ou lisse, par exemple il est en tissu, par exemple en un tissé polymère.
- le corps de sacoche est réalisé ou comprend des fibres polymère, typiquement un polyester.
- le panneau de protection est fixé de manière amovible au corps de sacoche, c'est-à-dire qu'il peut être détaché ou démonté du corps de sacoche, par exemple pour être remplacé s'il est abimé ou usé.
- le panneau de protection est fixé de manière amovible au niveau de la partie inférieure de la paroi avant du corps de sacoche, en particulier au niveau d'une paroi de fond du corps de sacoche, c'est-à-dire dans la zone ou région d'intersection entre le fond et la façade de la sacoche.
- le panneau de protection est fixé au corps de sacoche par des moyens de fixation formant charnière et assurant le pivotement de le panneau de protection par rapport au corps de sacoche autour d'un axe de pivotement (XX), typiquement un axe de pivotement horizontal.
- les moyens de fixation comprennent une fermeture à glissière (i.e. fermeture-éclair) agencée au niveau de la bordure périphérique de le panneau de protection et comprenant l'axe de pivotement (XX).
- le panneau de protection comprend en outre des premiers moyens de maintien configurés pour coopérer avec des seconds moyens de maintien agencés sur le corps de sacoche pour maintenir le panneau de protection face à la fenêtre principale lorsque ledit écran de protection est dans la position repliée.
- de préférence les premiers et les seconds moyens de maintien comprennent un système de fixation de type à bandes de type Velcro^{®}, i.e. une première partie de bande de type Velcro^{®} agencée sur le panneau de protection et une seconde partie de bande de type Velcro^{®} agencée sur corps de sacoche.
- le panneau de protection est mobile en pivotement entre la position repliée (P1) et au moins une position dépliée (P2) décalée angulairement de la position repliée (P1) d'au moins 80°, de préférence entre environ 80° et 210°, selon que la sacoche est en position debout ou couchée. Par exemple, les positions P1 et P2 sont décalés d'un angle de l'ordre de 90° lorsque la sacoche est en position debout ou d'un angle de l'ordre de 180° lorsque la sacoche est en position couchée.
- le panneau de protection est mobile en pivotement entre la position repliée (P1), la position dépliée (P2) et des positions angulaires intermédiaires situées ente lesdites positions repliée (P1) et dépliée (P2).
- le panneau de protection est sensiblement plat.
- en position repliée P1, les première et seconde vitres se font face, c'est-à-dire parallèles ou quasi-parallèles l'une à l'autre.
- en position repliée P1, les première et seconde vitres sont positionnées à faible distance l'une de l'autre, typiquement au contact ou quasi-contact l'une de l'autre.
- le corps de sacoche comprend la paroi avant, aussi appelée façade ou face avant, et par ailleurs une paroi de fond, une paroi arrière, une première paroi latérale, aussi appelée paroi latérale droite, une second paroi latérale, aussi appelée paroi latérale gauche, et une paroi de toit ou de dessus (lorsque la sacoche est considérée en position « debout », c'est-à-dire posée verticalement sur une surface, tel le sol).
- elle comprend un ventilateur médical logé dans le compartiment interne.
- elle comprend un sac à accessoires fixé au corps de sacoche.
- le sac à accessoires est détachable, c'est-à-dire qu'il peut être désolidarisé du corps de sacoche.
- les parois définissent entre elles le compartiment interne configuré et dimensionné pour loger un ventilateur médical, c'est-à-dire un volume interne apte à recevoir un ventilateur médical.
- la paroi avant, la paroi arrière et les deux parois latérales se projettent vers le haut en surmontant la paroi de fond.
- la paroi avant et la paroi arrière sont reliées entre elles par les deux parois latérales.
- la paroi avant fait face à la paroi arrière.
- les deux parois latérales sont agencées face à face.
- la paroi de toit surmonte la paroi avant, la paroi arrière et les deux parois latérales.
- la paroi de toit fait face à la paroi de fond.
- la paroi de fond et/ou la paroi arrière comprennent un revêtement externe antidérapant.
- la paroi de fond et/ou la paroi arrière sont renforcées, typiquement présentent une surépaisseur, c'est-à-dire comprennent un ou des éléments de renfort.
- elle est équipée d'une poignée de portage.
- la paroi de toit comprend une poignée de portage.
- la poignée de portage est de forme courbe ou arquée.
- la poignée de portage est texturée ou conformée pour ne pas glisser et/ou conférer une sensation agréable lorsque l'utilisateur la saisit à la main, i.e. manuellement.
- la poignée de portage est formée d'une âme centrale tubulaire entourée d'une gaine en tissu, de préférence recouverte d'un revêtement de caoutchouc ou analogue.
- le corps de sacoche comprend en outre des moyens d'ouverture disposés le long des intersections entre la paroi arrière et les deux parois latérales, en particulier des fermetures à glissière.
- la paroi de toit comprend un rabat venant recouvrir une partie de la paroi arrière.
- la paroi de fond comprend un élément de renfort interne permettant d'améliorer sa rigidité et/ou la stabilité de la sacoche lorsqu'elle est posée en position verticale.
- la paroi de fond comprend, i.e. est percée, des orifices d'évacuation des gaz contenus dans le compartiment interne, en particulier des orifices (i.e. trous) servant à évacuer les gaz expirés par un patient ventilé au moyen du ventilateur médical contenu dans la sacoche.
- le corps de sacoche comprend en outre des moyens de fixation pour y fixer un sac de rangement optionnel, appelé « sac à accessoires », servant à stocker des accessoires et/ou des consommables, tel que masque, tuyaux flexibles.... et/ou d'autres objets, par exemple un manuel d'utilisation ou autre.
- le sac à accessoires comprend un volume interne pour y ranger/stocker des accessoires et/ou des consommables, et un moyen d'ouverture/fermeture, telle une fermeture éclair ou analogue, permettant d'ouvrir ou fermer le sac pour donner accès ou interdire l'accès au volume interne du sac à accessoires.
- le sac à accessoires est agencé du côté de la face ou paroi arrière du corps de sacoche.
- le sac à accessoires est fixé au corps de sacoche par des moyens de fixation comprenant un ou plusieurs systèmes à boucle et mousqueton et/ou des connecteurs mâle/femelle, c'est-à-dire à raccordement par emboitement ou analogue.

L'invention concerne aussi un ensemble comprenant une sacoche selon l'invention, dans laquelle est inséré/logé un ventilateur médical, et par ailleurs un sac à accessoires fixé à ladite sacoche.

De plus, l'invention concerne aussi une utilisation d'une sacoche selon l'invention pour transporter un ventilateur médical, ledit ventilateur médical étant inséré ou logé dans ladite sacoche, en particulier dans le compartiment interne du corps de sacoche.

L'invention va maintenant être mieux comprise grâce à la description détaillée suivante, faite à titre illustratif mais non limitatif, en référence aux figures annexées parmi lesquelles :
Fig. 1 représente un mode de réalisation d'une sacoche selon l'invention, vue de face, avec un ventilateur médical inséré dans son compartiment interne mais sans le panneau de protection.
Fig. 2 montre la sacoche de Fig. 1 en position debout avec panneau de protection avant en position dépliée (P2).
Fig. 3 est analogue à Fig. 1 mais représente la sacoche vide, c'est-à-dire sans le ventilateur.
Fig. 4 est une vue de côté de la sacoche de Fig. 1.
Fig. 5 est analogue à Fig. 1 mais montre le ventilateur pendant son fonctionnement.
Fig. 6 est une vue de côté de la sacoche de Fig. 5 à laquelle est fixé un sac de rangement optionnel.
Fig. 7 est une vue de la sacoche de Fig. 1 en position couchée sur sa face arrière avec panneau de protection avant déployé.
Fig. 8 schématise le positionnement du sac à accessoires sous la sacoche de Fig. 1, lorsque la sacoche est fixé à un support.

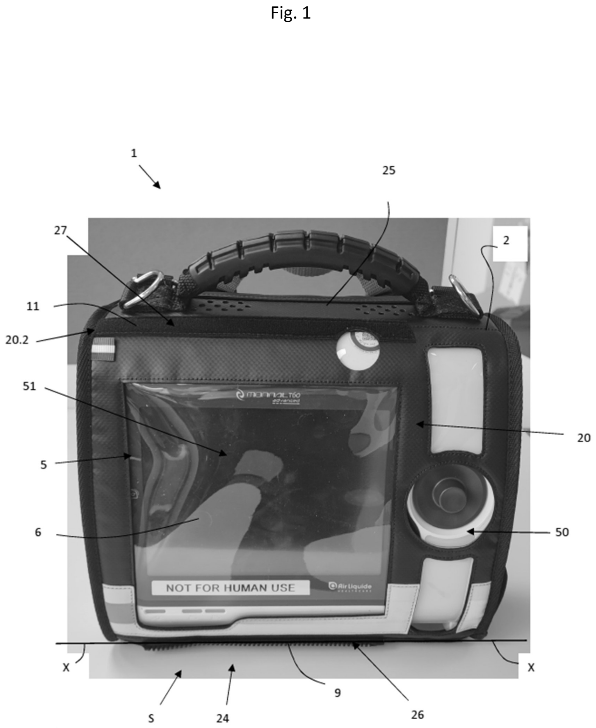

Fig. 1, Fig. 2 et Fig. 5 représentent un mode de réalisation d'une sacoche 1 de transport selon l'invention, vue de face, comprenant un ventilateur médical 50 inséré dans son compartiment interne 4.

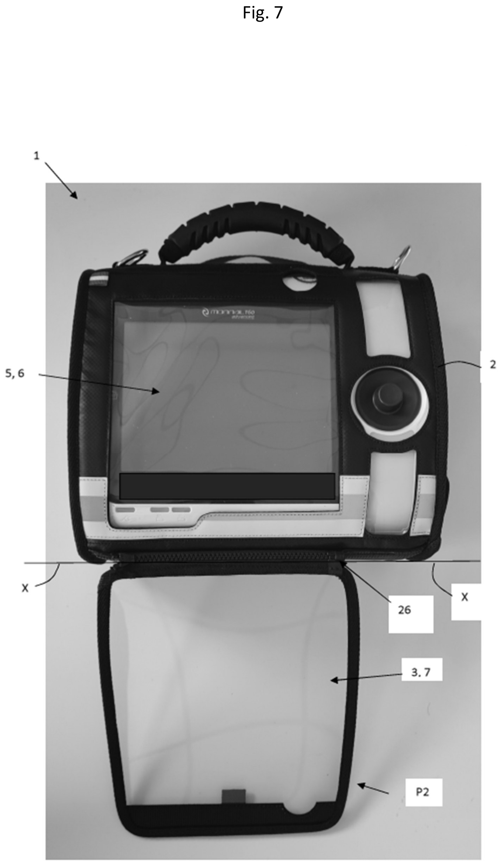

La sacoche 1 comprend un corps de sacoche 2, de préférence relativement souple, par exemple en un tissé de polymère ou analogue. Elle peut être utilisée en position « debout » comme illustré en Fig. 1 ou en position « couchée » comme illustré en Fig. 7, en particulier quand un ventilateur médical 50 y est logé.

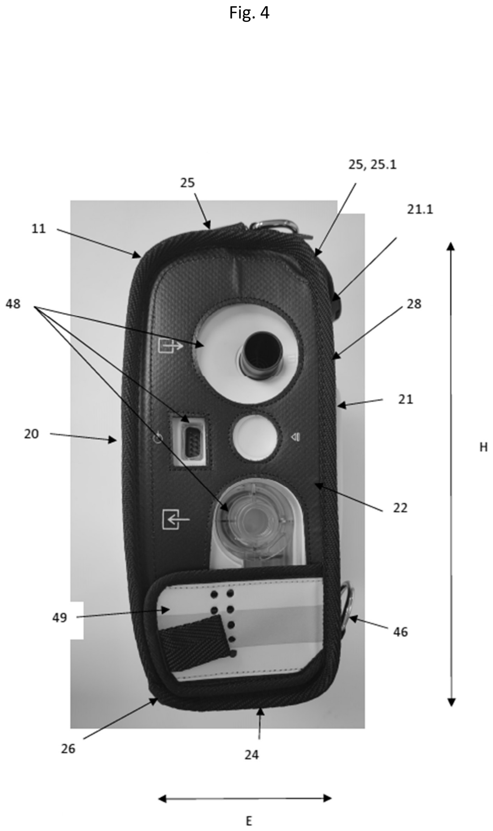

Comme visible sur Fig. 1 et Fig. 4, le corps de sacoche 2 comprend une paroi avant 20, aussi appelée face avant ou façade, une paroi de fond 24, une paroi arrière 21, une première paroi latérale 22, aussi appelée paroi latérale droite, une seconde paroi latérale 23, aussi appelée paroi latérale gauche, et une paroi de toit 25 ou de dessus, lorsque la sacoche 1 est considérée en position « debout », c'est-à-dire posée verticalement sur une surface S, tel le sol, une table ou autre, comme illustré sur Fig. 1 à Fig. 6. Les parois avant 20 et arrière 21 se font face, tout comme les parois latérales 22, 23, et les parois de fond 24 et de toit 25. Les parois 20-25 définissent entre elles le compartiment interne 4 qui est configuré et dimensionné pour loger le ventilateur médical 50. Le ventilateur médical 5 est introduit dans le compartiment interne 4 par le haut de la sacoche 1.

Préférentiellement, le corps de sacoche 2 a une largeur (L) comprise entre 30 et 50 cm, une hauteur (H) comprise entre 20 et 40 cm environ et une épaisseur (E) comprise entre 10 et 20 cm environ.

Afin de pouvoir utiliser le ventilateur médical 50, sans avoir à l'extraire du compartiment interne 4 de la sacoche 1 de transport, une fenêtre principale 5 est aménagée dans la paroi avant 20 du corps de sacoche 2, au sein de laquelle est agencé et/ou fixée une vitre transparente souple 6 (i.e. une première vitre), typiquement une feuille souple en polymère.

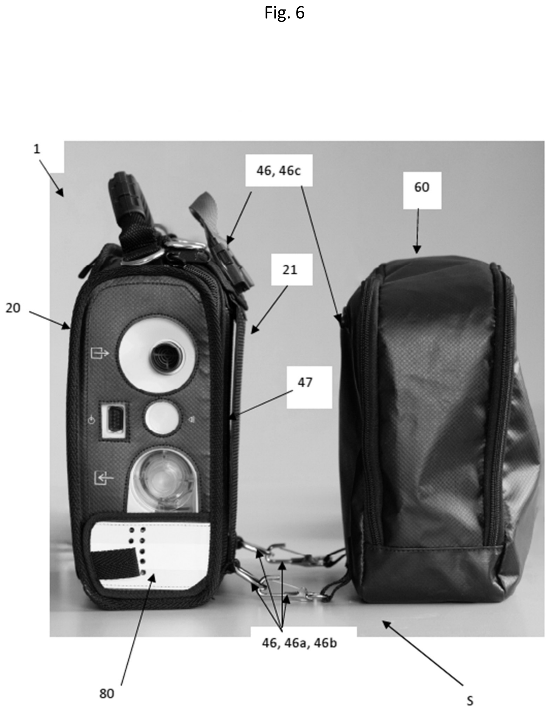

Sur Fig. 1, le ventilateur 50 est 'éteint', c'est-à-dire qu'il ne fonctionne pas, comme pendant un transport par exemple, alors que sur Fig. 6, le ventilateur 50 est 'allumé', c'est-à-dire qu'il fonctionne et affiches des informations 52 sur son moniteur 51.

La vitre transparente souple 6 vient recouvrir la fenêtre principale 5, c'est-à-dire obturer la découpe ou ouverture formant la fenêtre principale 5. Cette vitre transparente souple 6 est transparente pour permettre à un utilisateur, tel un personnel soignant (e.g. médecin...), de visualiser les indications et autres informations 52 affichées par le moniteur 51 du ventilateur médical 50, comme illustré en Fig. 6, typiquement un moniteur 51 à écran d'affichage tactile, de préférence en couleurs.

Par ailleurs, la vitre 6 ou feuille est souple, i.e. flexible, pour permettre à l'utilisateur d'interagir avec le moniteur 51 du ventilateur médical 50, en particulier lorsqu'il est à dalle tactile, c'est-à-dire que l'utilisateur peut opérer des sélections, des validations ou autres par appui digital sur la surface externe de la vitre souple 6, lesquels appuis se répercutent sur l'écran d'affichage du moniteur d'affichage 51 du ventilateur médical 50 et permettent d'opérer les choix, les sélections, les validations ou autres au sein des affichages 52 présents sur l'écran du moniteur 51, comme illustré en Fig. 6.

Cette vitre ou feuille transparente souple 6 permet aussi de protéger le moniteur 51 du ventilateur médical 50 contre les chocs, les salissures, les rayures ou autres agressions extérieures.

Par ailleurs, selon l'invention, la sacoche 1 comprend aussi un panneau de protection 3 mobile, i.e. pivotant ou basculant, formant une sorte de « bouclier » de protection avant. Le panneau de protection 3 comprend une vitre transparente rigide 7, typiquement une plaque en polymère transparente. Un rebord ou une bordure périphérique 30 est agencée autour de la seconde vitre transparente 7.

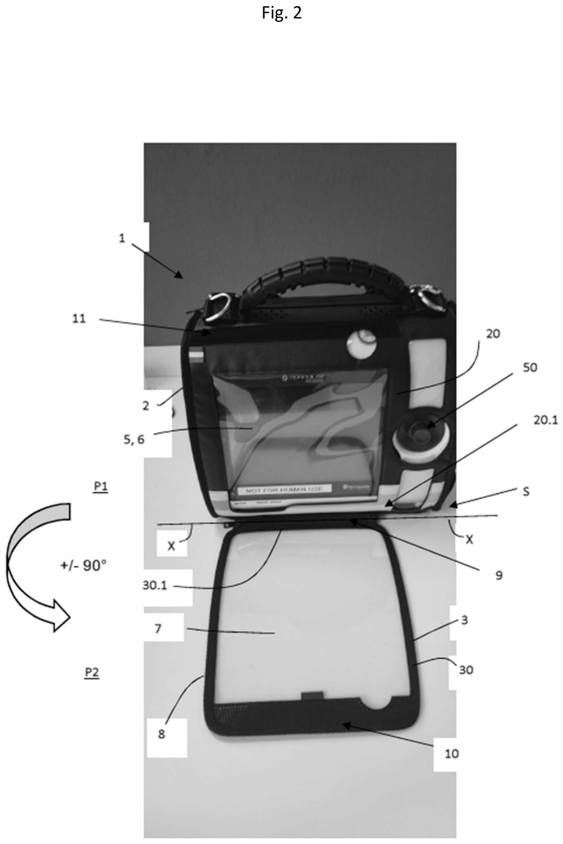

Le panneau 3 est fixé de manière pivotante, i.e. basculante, au corps de sacoche 2 entre au moins :
- une position repliée P1, dans laquelle le panneau de protection 3 vient se placer face à la paroi avant 20 de la sacoche, c'est-à-dire que la vitre rigide 7 et la vitre souple 6 de la fenêtre principale 5 sont agencées face à face, c'est-à-dire sensiblement parallèles l'une à l'autre, et
- une position dépliée P2, dans laquelle le panneau de protection 3 est déplacé angulairement, i.e. pivoté, par rapport à la paroi avant 20 de la sacoche, comme visible en Fig. 2.

Typiquement, pour passer de la position repliée P1 à la position dépliée P2 (ou inversement), le panneau de protection 3 est pivoté d'environ 80 à 100°, typiquement de l'ordre de 90°, lorsque la sacoche 1 est en position « debout », c'est-à-dire qu'elle est posée sur son fond 25, comme visible sur Fig. 2, et/ou d'environ 170 à 200°, typiquement de l'ordre de 180° à 190°, lorsque la sacoche 1 est en position « couchée », c'est-à-dire qu'elle est posée sur sa face arrière 21, avec panneau ou « bouclier » de protection avant 3 déployé, comme visible sur Fig. 7.

Bien entendu, il existe donc des positions angulaires intermédiaires que peut adopter le panneau 3 pendant son basculement/pivotement entre les positions P1 et P2, ou inversement.

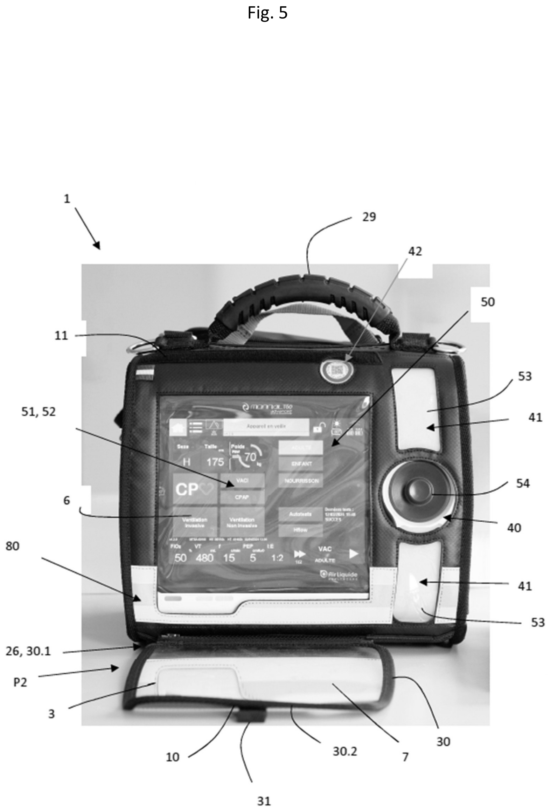

Le pivotement du panneau de protection 3 se fait autour d'un axe XX sensiblement horizontal (quand la sacoche 1 est posée sur une surface comme sur Fig. 5), lequel est situé au niveau de l'intersection entre le fond 24 et la face avant 20 de la sacoche, comme visible en Fig. 1.

Lorsqu'il est en position repliée P1, le panneau de protection 3 est destiné à venir protéger la vitre souple 6, i.e. première vitre, de la fenêtre principale 5, par exemple pendant le transport de la sacoche 1 équipée d'un ventilateur 50, pour éviter qu'elle ne soit détériorée. Dans ce cas, les vitres souple 6 et rigide 7 se font face, c'est-à-dire qu'elles sont positionnées sensiblement parallèles l'une de l'autre et au contact ou quasi-contact l'une de l'autre, i.e. situées à faible distance l'une de l'autre, par exemple à quelques millimètres au maximum.

La vitre rigide 7 étant transparente, elle permet à l'utilisateur de voir les affichages du moniteur 51, même lorsque le panneau 3 est en position P1, par exemple pour vérifier que la ventilation se déroule bien puisqu'il peut voir l'écran du moniteur 51 du ventilateur au travers des deux feuilles transparentes 6, 7. Toutefois, dans ce cas, la vitre rigide 7 empêche des appuis inopinés sur le moniteur 51 du ventilateur.

A l'inverse, lorsqu'il est en position dépliée P2, le panneau de protection 3 libère l'accès à l'utilisateur à la vitre souple 6 de la fenêtre principale 5 afin de permettre d'interagir avec le ventilateur 50 et le moniteur 51, comme susmentionné.

La première vitre transparente 6 est préférentiellement formée d'une feuille souple en polymère (plastique) de faible épaisseur pour lui conférer sa flexibilité, par exemple ayant une épaisseur de moins de 1 mm. De préférence, la vitre transparente souple 6 a une forme carrée ou rectangulaire, par exemple un carré de 18 cm x 18 cm ou de toutes autres dimensions adaptées.

A l'inverse, la seconde vitre transparente 7 est préférentiellement formée d'une feuille épaisse plus rigide, aussi en polymère (plastique), mais de plus forte épaisseur, typiquement une plaque pour lui conférer sa rigidité supérieure, par exemple d'au moins 1 mm. De préférence, la vitre rigide 7 a une forme carrée ou rectangulaire. Avantageusement ses dimensions peuvent être du même ordre ou équivalentes à celles de la première vitre souple 6, préférentiellement légèrement supérieures à celles de de la première vitre souple 6 afin de la recouvrir totalement, en position repliée P1, voire de déborder légèrement de chaque côté.

Le polymère formant la seconde vitre transparente rigide 7 est par exemple du PET, telle une plaque de PET, alors que celui formant la première vitre transparente est par exemple PVC, telle une feuille de PVC.

Le panneau de protection 3 comprenant la seconde vitre transparente rigide 7 est fixé de manière amovible au corps de sacoche 2, c'est-à-dire qu'il peut être démonté facilement afin de pouvoir, par exemple, le remplacer s'il est usé ou abimé.

Avantageusement, le panneau de protection 3 est fixé au niveau de la partie inférieure 20.1 de la paroi avant 20 du corps de sacoche 2, typiquement au niveau de l'intersection or bordure 26 entre la paroi avant 20 et le fond 24 de la sacoche 1.

A cette fin, on prévoit des moyens de fixation 9 formant charnière et assurant le pivotement de le panneau de protection 3 par rapport au corps de sacoche 2 autour de l'axe de pivotement (XX), typiquement une fermeture à glissière, i.e. fermeture-éclair ou « zip », agencée, d'une part, au niveau du bord inférieur 30.1 de la bordure périphérique 30 de le panneau de protection 3 et, d'autre part, en bas (20.1) de la paroi avant 20 de la sacoche 1, typiquement au niveau de l'intersection or bordure 26 entre la paroi avant 20 et le fond 24 de la sacoche 1.

Par ailleurs, le panneau de protection 3 comprend aussi des premiers moyens de maintien 10 configurés pour coopérer avec des seconds moyens de maintien 11 agencés sur le corps de sacoche 2, pour maintenir le panneau de protection 3 en position repliée P1, c'est-à-dire assurer un maintien du panneau de protection 3 face à la fenêtre principale 5, donc des deux vitres 6, 7 face à face.

Les premiers moyens de maintien 10 sont agencés au niveau du bord supérieur 30.2 de la bordure périphérique 30 de le panneau de protection 3.

Par ailleurs, les seconds moyens de maintien 11 sont agencés en haut 20.2 de la face avant 20 de la sacoche 1, typiquement au niveau de la zone ou région d'intersection 27 entre la paroi de toit 25 et la paroi ou face avant 20 de la sacoche 1, comme visibles en Fig. 1.

De préférence, les premiers moyens de maintien 10 et les seconds moyens de maintien 11 comprennent un système de fixation de type à bandes Velcro^{®} coopérant l'une avec l'autre. Bien entendu, un autre système de fixation pourrait convenir, comme des boutons-pression ou autres.

Comme visible en Fig. 5, le panneau de protection 3 comprend par ailleurs une languette 31 de préhension permettant à l'utilisateur de saisir le panneau avant 3 pour opérer son pivotement de la position P1 à la position P2 par exemple.

De plus, afin de faciliter le transport de la sacoche 1 et du ventilateur 50, la paroi de toit 25 est équipée d'une poignée de portage 29 configurée pour être saisie manuellement et facilement par un utilisateur, même lorsqu'il porte des gants. Avantageusement, la poignée 29 est de forme courbe ou arquée. Elle peut aussi être texturée ou conformée pour ne pas glisser et/ou conférer une sensation agréable lorsque l'utilisateur la saisit. Elle peut être formée par exemple d'une âme centrale tubulaire entourée d'une gaine en tissu venant notamment se fixer au toit 25, et recouverte d'un revêtement de caoutchouc ou analogue.

Afin de permettre d'insérer facilement le ventilateur 50 dans le compartiment interne 4 de la sacoche 1, typiquement par le haut de la sacoche 1, le corps de sacoche comprend en outre des moyens d'ouverture 28, telles des fermetures à glissière, disposés le long des intersections entre la paroi arrière 21 et les deux parois latérales 22, 23, i.e. les parois droite et gauche, de la sacoche 1.

De plus, la paroi de toit 25 comprend un rabat arrière 25.1 venant recouvrir une partie de la paroi arrière 21, c'est-à-dire le haut 21.1 de la paroi arrière 21, et pouvant être solidarisé à la paroi arrière 21 pour fermer la sacoche 1 ou, à l'inverse, détaché de la paroi arrière 21 afin de permettre notamment son ouverture et d'y insérer ou d'en extraire le ventilateur 50. Le système de maintien peut, là encore, un système de fixation de type à bandes Velcro^{®} coopérant l'une avec l'autre, l'une étant agencée en haut 21.1 de la paroi arrière 21 et l'autre étant agencée sur la face interne du rabat arrière 25.1.

Par ailleurs, la paroi de fond 24 comprend un (ou des) élément de renfort interne (non montré) permettant d'améliorer sa rigidité et la stabilité de la sacoche 1 lorsqu'elle est posée en position verticale, i.e. en position debout comme sur Fig. 1 ou Fig. 5. Selon un mode de réalisation, un ou des éléments de renfort peuvent aussi être prévus au niveau la paroi arrière 21.

Avantageusement, la paroi de fond 24 et/ou la paroi arrière 21 du corps 2 de sacoche peuvent comprendre un revêtement externe antidérapant.

En outre, la paroi de fond 24 est percée d'orifices d'évacuation des gaz contenus dans le compartiment interne 4, en particulier des orifices (i.e. trous) servant à évacuer les gaz expirés par un patient ventilé au moyen du ventilateur médical 50 contenu dans la sacoche 1 et qui sont habituellement retournés au ventilateur 1par une branche expiratoire d'un circuit patient reliant le ventilateur 5à à une interface respiratoire, tel un masque respiratoire, fournissant le gaz aux voies aériennes du patient.

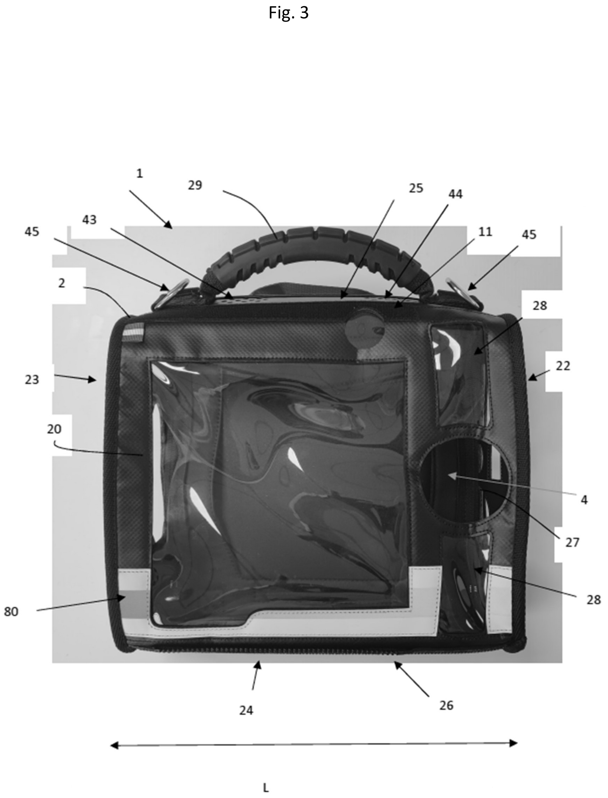

Avantageusement, la sacoche 1 peut aussi comprendre, d'une façon générale, d'autres éléments, notamment :
- sur façade ou paroi avant 20, comme illustré en Fig. 5 :
   ▪ une ouverture ou découpe 40, typiquement de forme circulaire, donnant accès à un bouton de réglage rotatif 54 du ventilateur 50,
   ▪ une ou plusieurs petites fenêtres additionnelles 41 (i.e. deux ici) équipées elles aussi d'une vitre transparente, telle une feuille en polymère souple analogue ou identique à celle de la première vitre 6, lesquelles permettent de visualiser, pendant l'utilisation du ventilateur 1, les dispositifs d'alarme lumineux 53 équipant le ventilateur 1. Elles se situent de part et d'autre, de préférence au-dessus et en dessous, de l'ouverture ou découpe 40 traversée par le bouton de réglage rotatif 54 du ventilateur 50.
   ▪ une fenêtre optionnelle 42 aussi transparente et munie d'une vitre souple en feuille de polymère, comme précédemment, pour permettre la lecture d'une étiquette de marquage, tel un QR code, un code-barres ou analogue, située sur le ventilateur 1.
- sur la paroi de toit 25, comme illustré en Fig. 3 :
   ▪ deux séries de trous ou orifices 43, 44, l'une pour rendre audible les alarmes sonores du ventilateur (i.e. buzzer) lorsqu'elles se déclenchent, l'autre pour permettre une évacuation des gaz expirés par le patient, en cas d'expiration via la valve de secours du ventilateur 1, en cas de panne ou analogue.
   ▪ une ou plusieurs paires de boucles métalliques 45 pour permettre d'y accrocher, d'une part, une bandoulière permettant un transport de la sacoche 1 à l'épaule par exemple et, d'autre part, des systèmes de fixation, par exemple des mousquetons ou analogue, pour suspendre la sacoche 1 avec le ventilateur 50 à un brancard, une réglette verticale dans un camion du SAMU ou sur un autre support.
- sur la face ou paroi arrière 21, comme illustré en Fig. 6 :
   ▪ des moyens de fixation 46 pour un sac de rangement 60 optionnel servant à stocker des accessoires et/ou des consommables, tel que masque, tuyaux flexibles... Par exemple, les moyens de fixation 46 peuvent comprendre un ou plusieurs systèmes à boucle 46a et mousqueton 46b et/ou des connecteurs mâle/femelle 46c, c'est-à-dire à raccordement par emboitement ou analogue.
   • un panneau central amovible 47, situé approximativement au milieu de la face arrière 21, pouvant être démonté en cas de besoin, typiquement pour donner accès à la partie arrière du ventilateur 50, en particulier lorsqu'il est équipé d'un accessoire de type platine VESA. Ceci permet de fixer le ventilateur 50 à un support mural par exemple sans avoir à retirer la sacoche 1 de protection au préalable. Ce panneau central amovible 47 peut être solidarisé au corps de sacoche 1 par des fixations de type Velcro^{®}.
- sur ses faces ou parois latérales 22, 23, i.e. droite et gauche, comme illustré en Fig. 4 :
   ▪ une ou des découpes supplémentaires 48 donnant accès à des éléments du ventilateur 50, tels que des prises de raccordement électriques ou pneumatiques (raccords...).
   ▪ un ou des petites portes 49 mobiles, notamment pivotantes, donnant accès elles aussi à des éléments du ventilateur 50, par exemple au compartiment à batterie ou analogue.

Sur Fig. 6, on voit un mode de réalisation, dans lequel les moyens de fixation 46 servant à fixer le sac de rangement 60 optionnel, aussi appelé sac à accessoires, comprenant :
- une paire de connecteurs mâle/femelle 46c à emboitement dont les éléments coopérant ensemble pour assurer la fixation (i.e. parties mâles et femelles des connecteurs) sont agencés en haut de la paroi arrière 21 de la sacoche 1 et en haut du corps du sac à accessoires 60, et
- une paire de systèmes de fixation 46 à boucle 46a et mousqueton 46b dont les éléments coopérant ensemble pour assurer la fixation (i.e. boucles et mousquetons) sont agencés en bas de la paroi arrière 21 de la sacoche 1 et en bas du corps du sac à accessoires 60.

Ainsi, le sac à accessoires 60 peut être fixé à la partie arrière 21 de la sacoche 1 et vient dès lors se positionner en regard de ladite partie arrière 21 de la sacoche 1, c'est-à-dire sensiblement en face et/ou parallèlement à l'un à l'autre.

Le sac à accessoire 60 peut être totalement détaché de la sacoche 1 par actionnement et libération/désolidarisation des moyens de fixation 46.

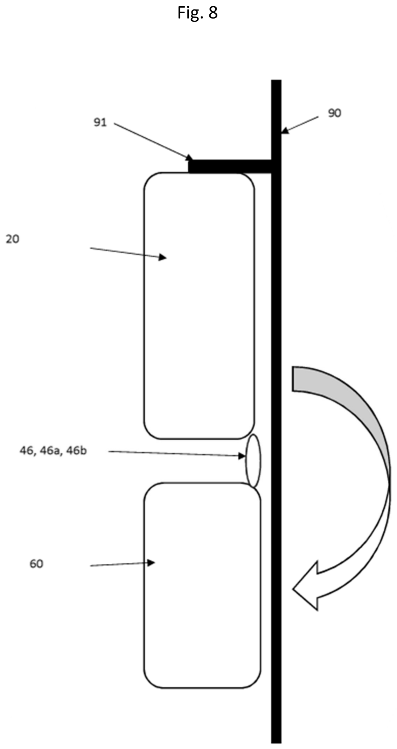

On peut aussi ne le détacher qu'en partie de la sacoche 1, par exemple en actionnant et libérant uniquement des moyens de fixation 46 situés en partie supérieure du sac à accessoires 60 et de la sacoche 1, à savoir la paire de connecteurs mâle/femelle 46c à emboitement de la Fig. 6. De cette façon, le sac à accessoires 60 peut « pivoter » autour d'un axe de pivotement passant sensiblement par les systèmes de fixation 46 à boucle 46a et mousqueton 46b situés en partie basse du sac 60 et de la sacoche 1. Comme illustré en Fig. 8, en effectuant un pivotement de l'ordre de 180°, le sac à accessoires 60 peut alors venir se placer en dessous de la sacoche 1. Ceci facilite le rangement de l'ensemble car cela permet de pouvoir accrocher la sacoche 1 le long d'une paroi 90, par exemple à un crochet 91 ou analogue, ou encore à une platine VESA, via la face arrière du ventilateur comme expliqué ci-avant, tout en conservant un accès aisé au sac à accessoires 60 puisqu'il vient alors se placer alors sous la sacoche 1. L'utilisateur peut donc accéder au contenu du sac à accessoires sans avoir besoin de manipuler ou décrocher la sacoche 1 de la platine VESA ou du crochet 91 mural.

Optionnellement, le corps de sacoche 1 peut porter des bandes réfléchissantes 80, par exemple en façade et/ou sur ses côtés droit et gauche.

D'une façon générale, une sacoche 1 de ventilateur médical 50 selon l'invention présente les avantages de :
- protéger le ventilateur 50 contre les salissures, rayures, l'humidité, les chocs...
- permettre un transport facile et sécurisé du ventilateur 50 et préférentiellement aussi des accessoires et consommables nécessaires à la ventilation (e.g. circuit patient, filtre, batterie secondaire...).
- offrir une bonne préhension grâce à sa poignée ergonomique.
- permettre à l'utilisateur d'interagir et/ou utiliser l'ensemble des interfaces, en particulier de l'écran d'affichage 51 du ventilateur 50, sans avoir à sortir le ventilateur 50 de la sacoche 1.,
- donner accès à la platine VESA arrière du ventilateur 50, sans avoir à le sortir de la sacoche 1.
- permettre à l'utilisateur de visualiser les informations affichées par l'écran d'affichage 51 du ventilateur 50, directement au travers de la vitre transparente 6 souple, lorsque le panneau avant 3 est en position dépliée P2, ou au travers des deux vitres 6, 7 lorsque le panneau avant 3 est en position repliée P1, donc là encore, sans avoir à l'extraire du compartiment interne 4 du ventilateur 50.
- permettre d'y accrocher un sac à accessoires optionnel.
- permettre à l'utilisateur d'accéder facilement au sac à accessoires, lorsqu'il est présent, même lorsque la sacoche est accrochée à une platine VESA, via le ventilateur, ou le long d'une paroi, sans avoir à décrocher la sacoche.

## Revendications

1. Sacoche de transport (1) pour ventilateur médical (50) comprenant un corps de sacoche (2) comprenant une paroi avant (20) et définissant un compartiment interne (4) configuré et dimensionné pour loger un ventilateur médical (50), dans laquelle la paroi avant (20) du corps de sacoche (2) comprend au moins une fenêtre principale (5) comprenant une première vitre (6) transparente, **caractérisée en ce qu'**un panneau de protection (3) comprenant une seconde vitre (7) transparente, est fixé de manière pivotante au corps de sacoche (2) pour venir se positionner face à la fenêtre principale (5) lorsqu'il est dans une position repliée (P1).

2. Sacoche selon la revendication 1, **caractérisée en ce que** la fenêtre principale (5) comprend une ouverture pratiquée dans la paroi avant (30) du corps de sacoche (2) et recouverte par la première vitre transparente (6).

3. Sacoche selon la revendication 1, **caractérisée en ce que** le panneau de protection (3) comprend une bordure périphérique (8) agencée autour de la seconde vitre transparente (7).

4. Sacoche selon la revendication 1, **caractérisée en ce que** la première vitre transparente et la seconde vitre transparente sont en polymère.

5. Sacoche selon la revendication 1, **caractérisée en ce que** le panneau de protection (3) est fixé de manière amovible au corps de sacoche (2).

6. Sacoche selon l'une des revendications 1 ou 5, **caractérisée en ce que** le panneau de protection (3) est fixé de manière amovible au niveau de la partie inférieure (20.1) de la paroi avant (20) du corps de sacoche (2).

7. Sacoche selon la revendication 1, 5 ou 6, **caractérisée en ce que** le panneau de protection (3) est fixé au corps de sacoche (2) par des moyens de fixation (9) formant charnière et assurant le pivotement du panneau de protection (3) par rapport au corps de sacoche (2) autour d'un axe de pivotement (XX), de préférence les moyens de fixation (9) comprennent une fermeture à glissière.

8. Sacoche selon l'une des revendications 1, 3 ou 5-7, **caractérisée en ce que** le panneau de protection (3) comprend en outre des premiers moyens de maintien (10) configurés pour coopérer avec des seconds moyens de maintien (11) agencés sur le corps de sacoche (2) pour maintenir le panneau de protection (3) face à la fenêtre principale (5) lorsque ledit le panneau de protection (3) est dans la position repliée (P1).

9. Sacoche selon la revendication 1, **caractérisée en ce que** le panneau de protection (3) est mobile en pivotement entre la position repliée (P1) et au moins une position dépliée (P2) décalée angulairement de la position repliée (P1) d'au moins 80°, de préférence entre environ 80° et 210°.

10. Sacoche selon l'une des revendications précédentes, **caractérisé en ce qu'**elle comprend un ventilateur médical (50) logé dans le compartiment interne (4) et/ou un sac à accessoires (60) fixé au corps (2) de sacoche.

11. Sacoche selon la revendication 1, **caractérisée en ce que** la première vitre transparente (6) est plus souple ou plus flexible que la seconde vitre transparente (7).

12. Sacoche selon la revendication 1, **caractérisée en ce qu'**elle est équipée d'une poignée de portage (29).

13. Sacoche selon la revendication 1, **caractérisée en ce que** la première vitre transparente (6) comprend un polymère de type polychlorure de vinyle (PVC) et/ou la seconde vitre transparente (7) comprend un polymère de type polytéréphtalate d'éthylène (PET).

14. Sacoche selon l'une des revendications 1 ou 13, **caractérisée en ce que** l'épaisseur de la première vitre transparente (6) est inférieure à l'épaisseur de la seconde vitre transparente (7).

15. Utilisation d'une sacoche selon l'une des revendications 1 à 14, pour transporter un ventilateur médical (50), ledit ventilateur médical (50) étant inséré ou logé dans le compartiment interne (4) du corps de sacoche (2).
